# EUROPEAN PATENT SPECIFICATION

(11) **EP 3 116 461 B1**
(45) Date of publication and mention of the grant of the patent: **27.12.2017**
(21) Application number: 15720809.1
(22) Date of filing: 10.03.2015
(51) Int. Cl.: A61G 7/075, A61G 13/12, A47C 20/00

(54) **POSTURAL DEVICE FOR THE CORRECT POSITIONING OF THE LOWER LIMBS OF BEDRIDDEN PATIENTS, AND MANUFACTURING PROCESS THEREOF**
HALTUNGSVORRICHTUNG FÜR DIE KORREKTE POSITIONIERUNG DER UNTEREN GLIEDMASSEN VON BETTLÄGERIGEN PATIENTEN UND HERSTELLUNGSVERFAHREN DAFÜR
DISPOSITIF POSTURAL POUR LE POSITIONNEMENT CORRECT DES MEMBRES INFÉRIEURS DE PATIENTS ALITÉS ET SON PROCÉDÉ DE FABRICATION

(30) Priority: 13.03.2014 IT BA20140017
(43) Date of publication of application: 18.01.2017
(73) Proprietor: Taccone, Luigi, 70013 Castellana Grotte (BA) (IT)
(72) Inventor: Taccone, Luigi, 70013 Castellana Grotte (BA) (IT)
(74) Representative: Fezzardi, Antonio
(86) International application number: PCT/IT2015/000065
(87) International publication number: WO 2015/136565

(56) References cited:
- KR-A- 20110 042 547
- US-B1- 6 272 703

## Description

The present invention regards a postural device for ensuring the correct position of the lower limbs of bedridden patients, as well as the corresponding industrial manufacturing process.

The invention presented herein is completely new, in so far as there currently do not exist similar devices for enabling optimal posture of the lower limbs of bedridden patients.

The disclosure referred to herein is likewise inventive in so far as the modifications made to known postural devices are such as to solve completely the problems linked to the posture of the lower limbs of bedridden patients.

Posturology is the scientific study that fundamentally regards the position of human beings both from the static standpoint and from the dynamic standpoint in the gravitational space occupied by the person's body, analysing various parameters such as his equilibrium, stature, stability or instability, together with the muscular contractions assumed by the parts of the body.

Analysis of posture can also determine the existence of clinical syndromes induced by a dysfunction of the postural system itself. These syndromes commonly fall under three distinct forms that are at times associated:
- painful postural syndromes, which mainly affect the spinal and muscolo-skeletal axis;
- instability and forms of dizziness of postural or phobic origin; and
- cognitive syndromes.

Posturologists have demonstrated that many health problems may frequently derive directly from postural problems that had previously been ignored or were difficult to diagnose.

For the above reasons, it may readily be understood how it is of fundamental importance to ensure the correct posture of bedridden patients, especially when the latter are in a comatose or vegetative state, namely in a state whereby any kind of autonomous movement is precluded, in particular movement of the lower limbs.

There currently exist a vast number of postural devices for correction of positioning of the lower limbs of patients.

US 6272703 B1 discloses a postural device of the related art. The disadvantages presented by traditional and known postural devices for the lower limbs of patients are basically due to the fact that, even though they may even be designed and built *ad hoc* for each individual bedridden patient, they do not always guarantee a perfect postural correction of the lower limbs. In particular, they have not so far enabled correction of forced extension of the knees of bedridden patients, and hence of the corresponding extra-rotation in the supine position of the lower limbs themselves on account of the prevalence of the gravitational forces acting on the lower limbs themselves with respect to the corresponding muscular forces, the latter being altogether absent in the case of patients with paralysed limbs.

In the same way, the prior art does not enable optimal correction of the stresses exerted on the vertebral column, especially in the lumbar region, thus acting in this region only partially or in a not altogether effective way.

A further disadvantage is constituted by the fact that the materials used for making cushions or known postural devices are not always optimal and even less do they enable or guarantee the possibility of carrying out perfect and convenient operations of periodic washing and hygienization.

The idea of improving current and known postural devices for bedridden patients arises from the need to enable assistants and relatives of the bedridden patient to overcome these disadvantages of the known art, as well as to be able to guarantee, via the use of the present improved postural device, the correct posture of the bedridden patient, in order to prevent any tendon and capsular-ligament retractions that might develop, if they are not prevented, into articular ankylosis, and hence in order to improve also the psycho-physical well-being of the bedridden patient.

The main purpose of the invention is in fact to optimise the conformation and corresponding functionality of current postural devices for bedridden patients, at the same time guaranteeing that they are made of materials that are easy to work and ensure a better hygienization.

A further purpose is in fact to provide postural devices that are improved as regards the materials making them up by adopting raw materials that are readily available and present a minimized cost but at the same time will guarantee both ease of hygienization of the postural device itself and ease of insertion of the latter behind the lower limbs of the bedridden patient.

Yet another purpose of the present invention is to provide a device constituted by a light material that can be easily moulded so that it can conveniently assume a shape conformable to the lower limbs of the body of the bedridden person who is to be sustained in a correct position.

The above purposes are achieved by providing a postural device 1 for the lower limbs 2 of bedridden patients, characterized in that it has a cylindrical shape 3 at its ends and a hemi-prismatic shape 4 in an intermediate section, this particular conformation of the device 1 being designed to enable the correct posture of a bedridden patient in a position with semi-flexion of the knees 6, it being possible for the knees 6 to rest in a recess P-Q-R-S, which bestows said hemi-prismatic section 4 on the device 1 and is designed, owing to the presence of the aforesaid cylindrical ends 3, to correct extra-rotation of the knees 6.

The above purposes and the consequent advantages, as well as the characteristics of the invention according to the present disclosure will emerge more clearly from the ensuing detailed description of a preferred solution, which is provided by way of non-limiting example, with reference to the annexed drawings, in which:

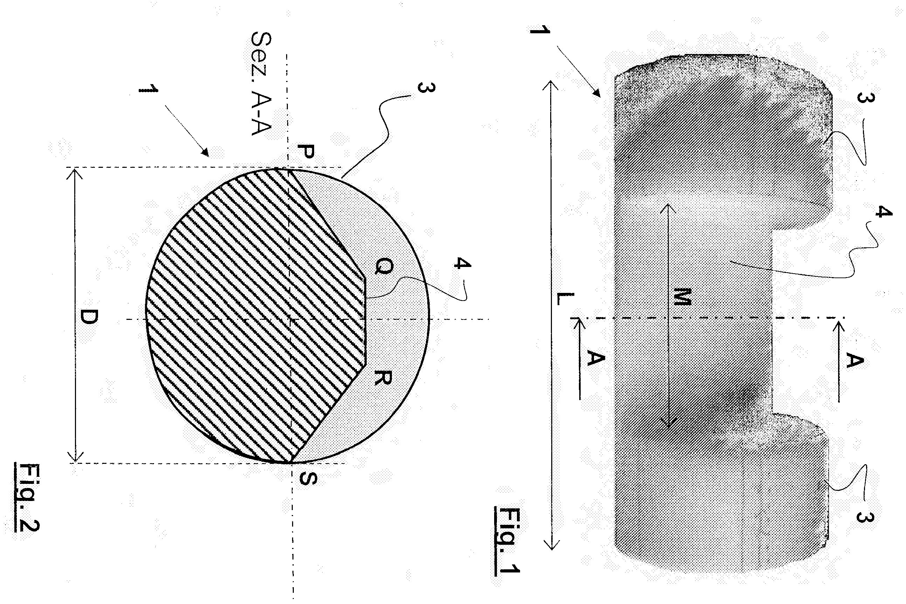

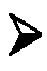 Figure 1 is a three-dimensional view of a preferred solution according to the present invention of a postural device 1 for the correction of the posture of the lower limbs 2 of bedridden patients, which has a particular cylindrical conformation 3 at its ends and is semi-cylindrical in an intermediate section; said cylindrical conformation 3 of the device 1 is designed to enable a slight rotation of the device 1 itself and hence a slight mobilisation in flexion-extension of the lower limbs 2 of the bedridden patient; from Figure1 it also emerges that, in the intermediate part, where the knees 6 of the bedridden patient are located and supported, the device 1 presents a recess with polygonal hemi-prismatic section 4;
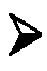 Figure 2 is a view of the cross section A-A indicated in Figure 1 of the aforesaid postural device 1 for the lower limbs 2 of bedridden patients, where there emerges said particular cylindrical conformation 3 of the device 1 interrupted in an intermediate portion by said hemi-prismatic recess 4, made in such a way as to enable a slight roll of the device 1 itself, when it is applied;
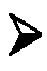 Figure 3 is a front view of the lower limbs 2 of a bedridden patient with evident extra-rotation in the supine position of the lower limbs 2, with forced extension of the knees 6;
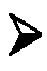 Figure 4 is a front view of the lower limbs 2 of a bedridden patient to whom the postural device 1 of Figure 1 has been applied;
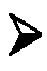 Figure 5 is a lateral view of the illustration of Figure 3;
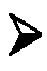 Figure 6 shows a diagram of a possible procedural sequence F of steps (F1-F2-F3-F4-F5-F6-F7) necessary for industrial manufacture of the aforesaid device 1; and
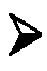 Figures 7 and 8, which are similar to Figures 1 and 2, show a variant in which, in the intermediate part, where the knees 6 of the bedridden patient are located and supported, the aforesaid device 1 is provided with a recess with curvilinear hemi-prismatic section 4.1 instead of a polygonal hemi-prismatic section 4.

As emerges from the attached Figures 1, 2, 4, and 5, the postural device 1 for bedridden patients 2 according to the present invention is basically constituted by a cushion made of foam rubber G or vinyl or other equivalent materials, having a substantially cylindrical shape 3, but provided with a recess with hemi-prismatic section 4, which functions as means for containing extra-rotation of the knees 6 and moreover as means for correcting forced extension of the lower limbs 2 of a bedridden patient.

The conformation of the above device 1 may be appreciated better from Figures 1 and 2.

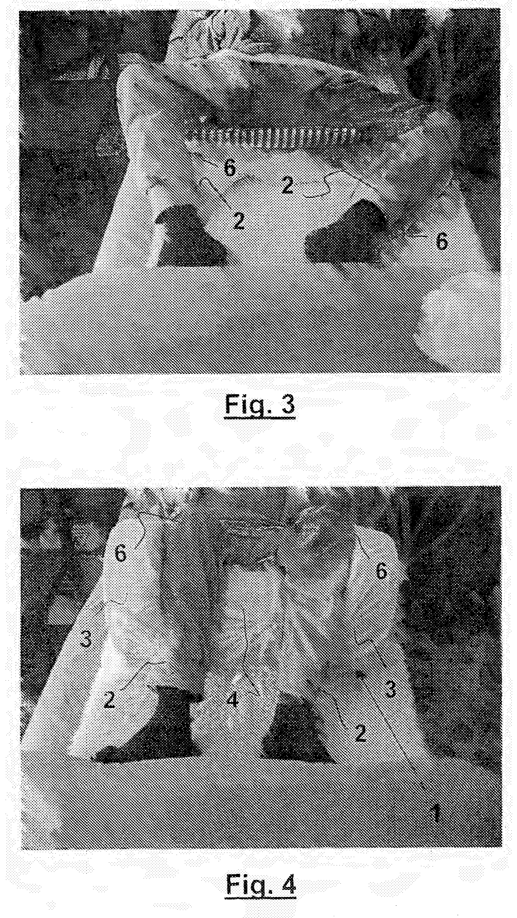

The modalities of insertion of the above postural device may be understood easily from the view of Figures 3, 4, and 5, where the device 1 appears correctly inserted under the lower limbs 2 of a bedridden patient.

The presence of the device 1 enables the bedridden patient to maintain a correct position of the lower limbs 2, in particular an alignment of the knees 6, which are prevented from performing extra-rotation, owing to the presence of a pair of cylindrical projections 3 at the ends of the device 1, as well as ensuring the correct posture in semi-flexion of the lower limbs 2 with the bedridden patient in the supine position.

The inventive step of the present device 1 is inherent in the fact of having devised an improvement of known postural devices, which affords the following advantages:
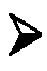 an optimal posture of the knees 6, as well as of the lower limbs 2 of the bedridden patient;
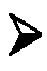 easy and inexpensive production given that it is constituted by a support that has a basically cylindrical shape 3 and is made of foam rubber or polyvinyl or other materials of equivalent elasticity and that afford ease of hygienization;
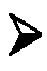 extreme lightness and just the right elasticity of the postural device 1 itself given that the latter is purposely made for each patient according to a given design for correcting the forced postures that are to be corrected or prevented; and
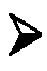 ease of hygienization of the device 1, in so far as it can be covered with an easily removable cover, which may be made of cotton or other materials that are readily available on the market and can be sterilized easily.

The postural device 1 for the lower limbs 2 of bedridden patients is likewise characterized in that it may be produced by cutting it to the desired shape manually at a handicraft level or even mechanically at an industrial level, in so far as it is constituted by a cylinder, obtained in particular starting from one of the aforesaid raw materials G that are readily available on the market and enable ease of hygienization. The cylinder usually has a diameter D of between 10 cm and 60 cm and a length L of between 20 cm and 100 cm. Formation of a recess P-Q-R-S subsequently bestows a characteristic hemi-prismatic section 4 on the device 1.

The aforementioned recess with hemi-prismatic section 4 is designed to provide a valid means of elastic support for the lower limbs 2 of the bedridden patient given that it is suited to correcting forced extension of the knees 6.

This recess, together with the presence of the afore-mentioned cylindrical ends 3 likewise constitutes a valid means for preventing extra-rotation of the knees 6 of the bedridden patient in the supine position. The above postural device 1 can likewise be easily covered with a cover made of cotton or other fabrics made of materials that enable ease of hygienization and are readily available on the market.

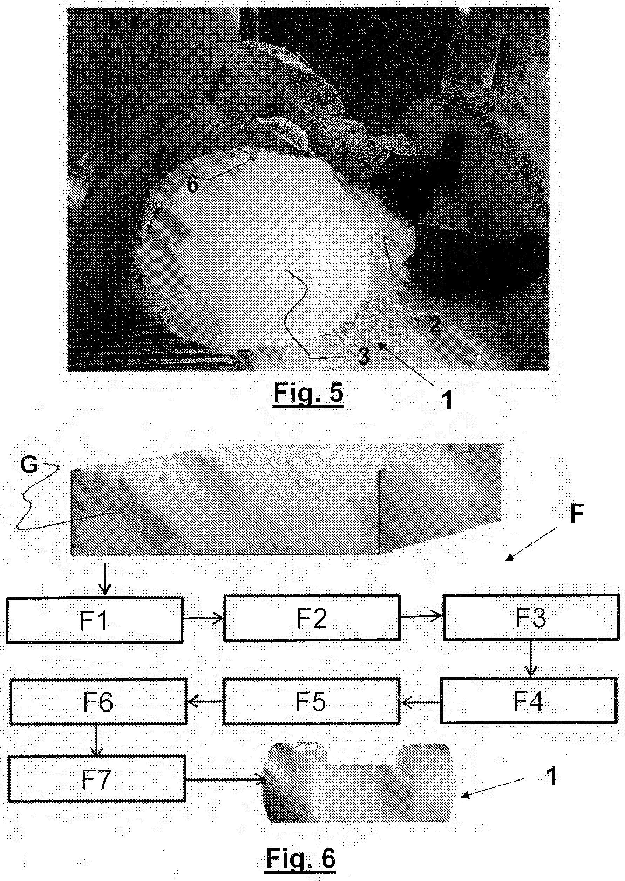

The device 1 can be easily produced according to the succession of the procedural steps, described by way of non-exhaustive example of a possible industrial manufacturing process F (Figure 6):
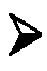 provision F1 of materials G that have a low density and can be easily hygienized;
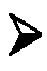 shaping F2 to form cylinders with quincuncial axes from the parallepipedal blocks of said materials G, according to design diameters D;
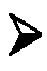 cutting F3 of the cylinders thus obtained to design lengths L;
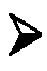 shaping F4 of a recess of width M, with a hemi-prismatic section 4, along a design polygonal line P-Q-R-S;
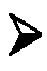 cleaning F5 of the device 1 thus obtained from processing residue and dust;
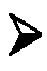 final hygienization F6 of the device 1;
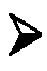 covering F7 of the device 1 with a possible cover made of cotton or other coating material that is readily available and enables ease of hygienization.

The above industrial manufacturing process F can also be obtained by producing first the intermediate section between the cylindrical ends 3 by connecting a hemi-prismatic section 4 to a hemi-cylindrical section 3 along their congruent rectangular bases, and then rigidly constraining a pair of cylindrical sections designed to provide said ends 3 at the coaxial ends of the intermediate section thus obtained.

The advantages deriving from the present invention are thus multiple and amply overcome the limitations of the known art, above all enabling a suitable postural device to be obtained that is altogether valid above all for bedridden patients. It is also evident that to the example of embodiment described previously by way of illustrative and non-limiting example numerous modifications, adaptations, integrations, variations may be made, as well as replacements of elements with other functionally equivalent ones, without thereby departing from the sphere of protection of the ensuing claims.

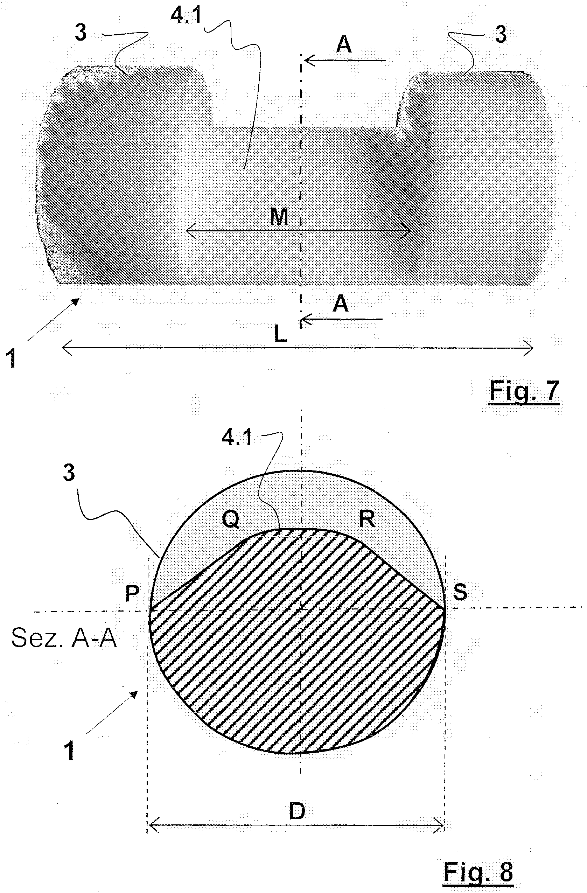

What has been described herein can be applied directly, without any modifications, also to the variant illustrated in Figures 7 and 8, where the aforesaid recess has a curvilinear hemi-prismatic section 4.1 instead of a polygonal hemi-prismatic section 4.

## Claims

1. A postural device (1) for the lower limbs (2) of bedridden patients, **characterized in that** it has a cylindrical conformation (3) at its ends and a hemi-prismatic conformation (4, 4.1) in an intermediate section, said particular conformation of the device (1) being designed to enable correct posture in semi-flexion of the knees (6), which would otherwise be forcedly extended, of a bedridden patient, the knees (6) being able to rest in a recess (P-Q-R-S) that bestows said hemi-prismatic section (4, 4.1) on the device (1) and is designed, owing to the presence of said cylindrical ends (3), to correct extra-rotation of the knees (6) themselves.

2. The postural device (1) for the lower limbs (2) of bedridden patients according to the preceding claim, **characterized in that** said cylindrical conformation (3) of the device (1) is designed to enable a slight rotation of the device (1) itself and hence a slight mobilization in flexion-extension of the lower limbs (2) of the bedridden patient.

3. The postural device (1) for the lower limbs (2) of bedridden patients according to any one of the preceding claims, **characterized in that** said cylindrical conformation (3) constitutes an unstable equilibrium of the device (1) that is able to provide optimal postural corrections.

4. The postural device (1) for the lower limbs (2) of bedridden patients according to any one of the preceding claims, **characterized in that** said postural device (1) is made of foam rubber or polyvinyl, or other materials (G) that enable ease of hygienization.

5. The postural device (1) for the lower limbs (2) of bedridden patients according to any one of the preceding claims, **characterized in that** said device (1) is obtained by cutting out manually or automatically a cylinder, which is made of one of said materials (G) that enable ease of hygienization and has a diameter (D) comprised between 10 cm and 60 cm and a length (L) comprised between 20 cm and 100 cm, as well as a recess that develops in cross section along a polygonal line (P-Q-R-S) and has a width (M), thus bestowing said hemi-prismatic section (4) on the

6. The postural device (1) for the lower limbs (2) of bedridden patients according to any one of the preceding claims, **characterized in that** said recess with hemi-prismatic section (4) is designed to provide a valid means of elastic support for the lower limbs (2) of the bedridden patient designed for correcting forced extension of the knees (6), as well as, via the presence of said cylindrical ends (3), constituting a valid means of prevention of extra-rotation of the knees (6) of the bedridden patient in the supine position.

7. The postural device (1) for the lower limbs (2) of bedridden patients according to any one of the preceding claims, **characterized in that** said postural device (1) is designed to be able to be easily covered with a cover made of cotton or other fabrics made of materials (G) that enable ease of hygienization.

8. The postural device (1) for the lower limbs (2) of bedridden patients according to any one of the preceding claims, **characterized in that** said hemi-prismatic section of said intermediate part is a polygonal hemi-prismatic section (4), or a curvilinear hemi-prismatic section (4.1).

9. An industrial manufacturing process (F) for the production of postural devices (1) for the lower limbs (2) of bedridden patients, **characterized by** the succession of the following production steps:
- provision (F1) of raw materials (G), which have a low density and enable ease of hygienization;
- shaping (F2) to form cylinders with quincuncial axes from the parallepipedal blocks of said raw materials (G), according to design diameters (D);
- cutting (F3) of the cylinders thus obtained according to the design lengths (L);
- shaping (F4) of a recess of a width (M), having a polygonal hemi-prismatic section (4) or a curvilinear hemi-prismatic section (4.1), along a design polygonal or curvilinear line (P-Q-R-S);
- complete cleaning (F5) from the processing residue and dust of the device (1) thus obtained;
- final hygienization (F6) of the device (1);
- covering (F7) of the device (1) with a possible cover made of cotton or some other material that is readily available on the market and affords ease of hygienization.

10. An industrial manufacturing process (F) for the production of postural devices (1) for the lower limbs (2) of bedridden patients, **characterized by** the succession of the following production steps:
- provision (F1) of raw materials (G), which have a low density and enable ease of hygienization;
- connecting a polygonal hemi-prismatic section (4) or a curvilinear hemi-prismatic section (4.1) to a hemi-cylindrical section (3) along their congruent rectangular bases
- rigidly constraining a pair of cylindrical sections designed to provide said cylindrical ends (3) at the coaxial ends of the intermediate section thus obtained;
- complete cleaning (F5) from the processing residue and dust of the device (1) thus obtained;
- final hygienization (F6) of the device (1);
- covering (F7) of the device (1) with a possible cover made of cotton or some other material that is readily available on the market and affords ease of hygienization.

## Patentansprüche

1. Haltungsvorrichtung (1) für die unteren Gliedmaßen (2) von bettlägerigen Patienten, **dadurch gekennzeichnet, dass** sie eine zylindrische Angleichung (3) an ihren Enden und
eine halbprismatische Angleichung (4, 4.1) in einem dazwischenliegenden Abschnitt aufweist,
wobei die besondere Angleichung der Vorrichtung (1) ausgebildet ist, eine korrekte Haltung bei Semiflexion der Kniee (6) eines bettlägerigen Patienten zu ermöglichen, die andererseits zwangsweise gestreckt werden würden, wobei die Kniee (6) in einer Ausnehmung (P-Q-R-S) ruhen können, die den halbprismatischen Abschnitt (4, 4.1) auf der Vorrichtung (1) verleiht und ausgebildet ist, wegen des Vorhandenseins der zylindrischen Enden (3), eine zusätzliche Drehung der Kniee (6) selbst zu korrigieren.

2. Haltungsvorrichtung (1) für die unteren Gliedmaßen (2) von bettlägerigen Patienten nach dem vorhergehenden Anspruch, **dadurch gekennzeichnet, dass** die zylindrische Angleichung (3) der Vorrichtung (1) ausgebildet ist, eine leichte Drehung der Vorrichtung (1) selbst und daher eine leichte Beweglichkeit bei Biegung-Streckung der unteren Gliedmaßen (2) des bettlägerigen Patienten zu ermöglichen.

3. Haltungsvorrichtung (1) für die unteren Gliedmaßen (2) von bettlägerigen Patienten nach einem der vorhergehenden Ansprüche, **dadurch gekennzeichnet, dass** die zylindrische Angleichung (3) ein labiles Gleichgewicht der Vorrichtung (1) bildet, das optimale Haltungskorrekturen bewirken kann.

4. Haltungsvorrichtung (1) für die unteren Gliedmaßen (2) von bettlägerigen Patienten nach einem der vorhergehenden Ansprüche, **dadurch gekennzeichnet, dass** die Haltungsvorrichtung (1) aus Schaumgummi oder Polyvinyl oder anderen Werkstoffen (G) besteht, die eine bessere Schaffung hygienischer Bedingungen ermöglichen.

5. Haltungsvorrichtung (1) für die unteren Gliedmaßen (2) von bettlägerigen Patienten nach einem der vorhergehenden Ansprüche, **dadurch gekennzeichnet, dass** die Vorrichtung (1) erhalten wird, indem ein Zylinder, der aus einem der Werkstoffe (G) hergestellt ist, die eine bessere Schaffung hygienischer Bedingungen ermöglichen, manuell oder automatisch ausgeschnitten wird und einen Durchmesser (D), der zwischen 10 cm und 60 cm liegt, und eine Länge (L) besitzt, die zwischen 20 cm und 100 cm liegt, sowie eine Ausnehmung aufweist, die sich im Querschnitt längs einer Polygonlinie (P-Q-R-S) ausbildet und eine Breite (M) aufweist, die somit den halbprismatischen Querschnitt (4) darauf verleiht.

6. Haltungsvorrichtung (1) für die unteren Gliedmaßen (2) von bettlägerigen Patienten nach einem der vorhergehenden Ansprüche, **dadurch gekennzeichnet, dass** die Ausnehmung mit halbprismatischem Querschnitt (4) ausgebildet ist, eine zulässiges Mittel elastischer Unterstützung für die unteren Gliedmaßen (2) des bettlägerigen Patienten bereitzustellen, gestaltet, um eine erzwungene Dehnung der Kniee (6) zu korrigieren, sowie über das Vorhandensein der zylindrischen Enden (3) ein zulässiges Mittel zur Verhinderung einer zusätzlichen Drehung der Kniee (6) des bettlägerigen Patienten in der Rückenlage zu bilden.

7. Haltungsvorrichtung (1) für die unteren Gliedmaßen (2) von bettlägerigen Patienten nach einem der vorhergehenden Ansprüche, **dadurch gekennzeichnet, dass** die Haltungsvorrichtung (1) ausgebildet ist, mit einer Umhüllung leicht bezogen werden zu können, die aus Baumwolle oder anderen Geweben hergestellt ist, die aus Materialien (G) bestehen, die eine bessere Schaffung hygienischer Bedingungen ermöglichen.

8. Haltungsvorrichtung (1) für die unteren Gliedmaßen (2) von bettlägerigen Patienten nach einem der vorhergehenden Ansprüche, **dadurch gekennzeichnet, dass** der halbprismatische Querschnitt des dazwischenliegenden Teils ein vieleckiger, halbprismatischer Querschnitt (4) oder ein krummlinig begrenzter, halbprismatischer Querschnitt (4.1) ist.

9. Industrielles Fertigungsverfahren (F) für die Produktion von Haltungsvorrichtungen (1) für die unteren Gliedmaßen (2) von bettlägerigen Patienten, **gekennzeichnet durch** die Serie der folgenden Produktionsschritte:
- Bereitstellung (F1) von Ausgangsmaterialien (G), die eine geringe Dichte besitzen und eine bessere Schaffung hygienischer Bedingungen ermöglichen;
- Formen (F2) zur Bildung von Zylindern mit Kreuzungsachsen aus den quaderförmigen Blöcken der Ausgangsmaterialien (G) entsprechend Entwurfsdurchmessern (D);
- Schneiden (F3) der so erhaltenen Zylinder entsprechend den Entwurfslängen (L);
- Formen (F4) einer Ausnehmung mit einer Breite (M), die einen vieleckigen, halbprismatischen Querschnitt (4) oder einen krummlinig begrenzten, halbprismatischen Querschnitt (4.1) entlang einer vieleckigen oder krummlinig begrenzten Linie (P-Q-R-S) besitzt;
- vollständiges Reinigen (F5) von Verarbeitungsrückstanden und Staub der so erhaltenen Vorrichtung (1);
- abschließende Schaffung hygienischer Bedingungen (F6) der Vorrichtung (1);
- Beziehen (F7) der Vorrichtung (1) mit einer möglichen Umhüllung, die aus Baumwolle oder irgendeinem anderen Material besteht, das ohne weiteres auf dem Markt erhältlich ist und eine bessere Schaffung hygienischer Bedingungen bewirkt.

10. Industrielles Fertigungsverfahren (F) für die Produktion von Haltungsvorrichtungen (1) für die unteren Gliedmaßen (2) von bettlägerigen Patienten, **gekennzeichnet durch** die Serie der folgenden Produktionsschritte:
- Bereitstellung (F1) von Ausgangsmaterialien (G), die eine geringe Dichte besitzen und eine bessere Schaffung hygienischer Bedingungen ermöglichen;
- Verbinden eines vieleckigen, halbprismatischen Querschnitts (4) oder eines krummlinig begrenzten, halbprismatischen Querschnitts (4.1) mit einem halbzylindrischen Querschnitt (3) entlang ihrer kongruenten, rechtwinkligen Grundflächen;
- starres Zusammenpressen eines Paars zylindrischer Querschnitte, die ausgebildet sind, die zylindrischen Enden (3) an den koaxialen Enden des so erhaltenen, dazwischenliegenden Querschnitts bereitzustellen;
- vollständiges Reinigen (F5) von Verarbeitungsrückstanden und Staub der so erhaltenen Vorrichtung (1);
- abschließende Schaffung hygienischer Bedingungen (F6) der Vorrichtung (1);
- Beziehen (F7) der Vorrichtung (1) mit einer möglichen Umhüllung, die aus Baumwolle oder irgendeinem anderen Material besteht, das ohne weiteres auf dem Markt erhältlich ist und eine bessere Schaffung hygienischer Bedingungen bewirkt.

## Revendications

1. Dispositif postural (1) pour les membres inférieurs (2) de patients alités, **caractérisé en ce qu'**il a une conformation cylindrique (3) à ses extrémités et une conformation hémi-prismatique (4, 4.1) dans une section intermédiaire, ladite conformation particulière du dispositif (1) étant conçue pour permettre une posture correcte en semi-flexion des genoux (6), qui seraient autrement forcément étendus, d'un patient alité, les genoux (6) étant capables de reposer dans un évidement (P-Q-R-S) qui confère ladite section hémi-prismatique (4, 4.1) sur le dispositif (1) et est conçu, en raison de la présence desdites extrémités cylindriques (3), pour corriger une rotation supplémentaire des genoux (6) eux-mêmes.

2. Dispositif postural (1) pour les membres inférieurs (2) de patients alités selon la revendication précédente, **caractérisé en ce que** ladite conformation cylindrique (3) du dispositif (1) est conçue pour permettre une légère rotation du dispositif (1) lui-même et par conséquent une légère mobilisation en flexion-extension des membres inférieurs (2) du patient alité.

3. Dispositif postural (1) pour les membres inférieurs (2) de patients alités selon l'une quelconque des revendications précédentes, **caractérisé en ce que** ladite conformation cylindrique (3) constitue un équilibre instable du dispositif (1) qui est en mesure de fournir des corrections posturales optimales.

4. Dispositif postural (1) pour les membres inférieurs (2) de patients alités selon l'une quelconque des revendications précédentes, **caractérisé en ce que** ledit dispositif postural (1) est réalisé en caoutchouc ou polyvinyle ou d'autres matériaux (G) qui permettent une facilité d'hygiénisation.

5. Dispositif postural (1) pour les membres inférieurs (2) de patients alités selon l'une quelconque des revendications précédentes, **caractérisé en ce que** ledit dispositif (1) est obtenu en découpant manuellement ou de manière automatique un cylindre qui est réalisé en un desdits matériaux (G) qui permettent une facilité d'hygiénisation et a un diamètre (D) compris entre 10 cm et 60 cm et une longueur (L) comprise entre 20 cm et 100 cm, ainsi qu'un évidement qui se développe en section transversale suivant une ligne polygonale (P-Q-R-S) et a une largeur (M), en conférant ainsi ladite section hémi-prismatique (4) sur le...

6. Dispositif postural (1) pour les membres inférieurs (2) de patients alités selon l'une quelconque des revendications précédentes, **caractérisé en ce que** ledit évidement avec la section hémi-prismatique (4) est conçu pour fournir un moyen valide de support élastique pour les membres inférieurs (2) du patient alité, conçu pour corriger une extension forcée des genoux (6), ainsi que, par la présence desdites extrémités cylindriques (3), constituant un moyen valide de prévention d'une rotation supplémentaire des genoux (6) du patient alité dans la position couchée sur le dos.

7. Dispositif postural (1) pour les membres inférieurs (2) de patients alités selon l'une quelconque des revendications précédentes, **caractérisé en ce que** ledit dispositif postural (1) est conçu pour pouvoir être facilement revêtu avec un revêtement réalisé en coton ou d'autres tissus constitués de matériaux (G) qui permettent une facilité d'hygiénisation.

8. Dispositif postural (1) pour les membres inférieurs (2) de patients alités selon l'une quelconque des revendications précédentes, **caractérisé en ce que** ladite section hémi-prismatique de ladite partie intermédiaire est une section hémi-prismatique polygonale (4) ou une section hémi-prismatique curviligne (4.1).

9. Processus de fabrication industriel (DF pour la production de dispositifs posturaux (1) pour les membres inférieurs (2) de patients alités, **caractérisé par** la succession des étapes de production suivantes :
- la fourniture (F1) de matériaux bruts (G) qui ont une faible densité et permettent une facilité d'hygiénisation ;
- le modelage (F2) pour former des cylindres avec des axes en quinconce à partir de blocs parallélépipèdes desdits matériaux bruts (G), en fonction de diamètres de conception (D) ;
- la découpe (F3) des cylindres ainsi obtenus en fonction des longueurs de conception (L) ;
- le modelage (F4) d'un évidement d'une largeur (M), ayant une section hémi-prismatique polygonale (4) ou une section hémi-prismatique curviligne (4.1), suivant une ligne polygonale ou curviligne de conception (P-Q-R-S) ;
- le nettoyage complet (F5) des résidus et de la poussière de traitement du dispositif (1) ainsi obtenu ;
- l'hygiénisation finale (F6) du dispositif (1) ;
- le revêtement (F7) du dispositif (1) avec un éventuel revêtement constitué de coton ou un certain autre matériau qui est facilement disponible sur le marché et assure une facilité d'hygiénisation.

10. Processus de fabrication industriel (F) pour la production de dispositifs posturaux (1) pour les membres inférieurs (2) de patients alités, **caractérisé par** la succession des étapes de production suivantes :
- la fourniture (F1) de matériaux bruts (G) qui ont une faible densité et permettent une facilité d'hygiénisation ;
- la connexion d'une section hémi-prismatique polygonale (4) ou d'une section hémi-prismatique curviligne (4.1) à une section demi-cylindrique (3) suivant leurs bases rectangulaires congruentes
- la fixation rigide d'une paire de sections cylindriques conçues pour fournir lesdites extrémités cylindriques (3) au niveau des extrémités axiales de la section intermédiaire ainsi obtenue ;
- le nettoyage complet (F5) des résidus et de la poussière de traitement du dispositif (1) ainsi obtenu ;
- l'hygiénisation finale (F6) du dispositif (1) ;
- le revêtement (F7) du dispositif (1) avec un éventuel revêtement constitué de coton ou un certain autre matériau qui est facilement disponible sur le marché et assure une facilité d'hygiénisation.
